# EUROPEAN PATENT APPLICATION

(11) **EP 1 439 158 A1**
(43) Date of publication of application: **21.07.2004**
(21) Application number: 02777926.3
(22) Date of filing: 22.10.2002
(51) Int. Cl.: C07C 27/02, C07C 69/96, C07C 68/06, C07C 68/08, C07C 37/52, C07C 39/15, C08J 11/10, C08G 64/42

(54) **METHOD OF DEPOLYMERIZING AROMATIC POLYCARBONATE**

(30) Priority: 26.10.2001 JP 2001328739; 26.10.2001 JP 2001328742; 26.10.2001 JP 2001328743
(71) Applicant: TEIJIN LIMITED, Osaka-shi Osaka 541-0054 (JP)
(72) Inventor: BAN, Tetsuo, c/o Teijin Limited, Iwakuni-shi, Yamaguchi 740-0014 (JP); YOSHISATO, Eishin, c/o Teijin Limited, Iwakuni-shi, Yamaguchi 740-0014 (JP); MURAMOTO, Masaharu, c/o Teijin Limited, Iwakuni-shi, Yamaguchi740-0014 (JP); OHASHI, Kenji, Teijin Limited, Iwakuni-shi, Yamaguchi 740-0014 (JP); HIRATA, Masumi, c/o Teijin Limited, Iwakuni-shi, Yamaguchi 740-0014 (JP)
(74) Representative: HOFFMANN - EITLE
(86) International application number: PCT/JP2002/010950
(87) International publication number: WO 2003/035592

(57) **Abstract**

An aromatic polycarbonate depolymerization method comprising reacting an aromatic polycarbonate with water, an aliphatic alcohol having 1 to 6 carbon atoms or a phenol which may be substituted by a hydrocarbon group having 1 to 10 carbon atoms in the critical fluid of carbon dioxide or with the critical fluid of an aliphatic alcohol having 1 to 6 carbon atoms or a phenol which may be substituted by a hydrocarbon group having 1 to 10 carbon atoms.

With this depolymerization method, the aromatic polycarbonate is depolymerized at a high reaction rate and effective components formed by depolyemrization can be recovered at a high rate. Therefore, the effective components are recovered from the scrapped or used aromatic polycarbonate by this method and recycled.

## Description

### Field of the Invention

The present invention relates to a method for depolymerization of an aromatic polycarbonate. More specifically, it relates to a method of depolymerizing an aromatic polycarbonate to recover effective components such as an aromatic bisphenol.

### Description of the Prior Art

Aromatic polycarbonate resins are materials of extremely high added value used for various applications such as lenses, compact disks, construction materials, auto parts, the chassis of OA equipment and camera bodies because they have high transparency, excellent optical properties and strong physical properties. Therefore, demand for the above resins is growing. After these products are used, most of them are burnt or buried in the ground as waste. This causes not only the waste of resources but also global-scale social problems such as the pollution of environment and the discharge of carbonic acid gas. Therefore, a so-called chemical recycling method for depolymerizing an aromatic polycarbonate resin which becomes waste and recycling it as a monomer is strongly desired. However, several methods have been proposed up till now but an effective method has yet to be found.

As for the depolymerization reaction of an aromatic polycarbonate, a method of hydrolyzing an aromatic polycarbonate in the presence of a nitrogen-containing compound such as ammonia water to obtain bisphenol A is proposed by JP-B 39-19159 (the term "JP-B" as used herein means an "examined Japanese patent publication"), a method of reacting an aromatic polycarbonate with an alcohol in the presence of an ester exchange catalyst to obtain bisphenol A and a carbonic acid diester corresponding to the used alcohol is proposed by JP-B 39-28648, a method of hydrolyzing an aromatic polycarbonate using an alkali aqueous solution to obtain bisphenol A is proposed by JP-B 40-16536, a method of depolymerizing an aromatic polycarbonate using a polar neutral solvent such as dimethyl sulfoxide and a hydroxy compound is proposed by JP-A 4-505930 (the term "JP-A" as used herein means an "unexamined published Japanese patent application"), and a method of subjecting an aromatic polycarbonate to an ester exchange reaction with a phenol to form a bisphenol and a diaryl carbonate is proposed by JP-A 6-56985. However, these methods have a defect that a relatively large amount of an ester exchange catalyst such as an alkali is required as the depolymerization rate is low with the result that the recycling, neutralization and the like of the catalyst become complicated, thereby greatly reducing economic efficiency in most cases. In the case of depolymerization using a phenol, the depolymerization rate is low, it is difficult to separate a bisphenol from a diaryl carbonate, and a repolymerization reaction is caused by the removal of the phenol when the phenol is distilled out too much by thermal separation. Therefore, as means of separating the bisphenol from the diaryl carbonate while an excessive amount of the residual phenol is left in the reaction product, it is proposed that an adduct of the bisphenol and the phenol in a ratio of 1:1 is crystallized and separated by controlling the amount of the residual phenol and then the diaryl carbonate is flash distilled out from the mother liquor. However, the diaryl carbonate itself tends to form an adduct with the phenol in a ratio of 1:1 and cannot be separated completely. To obtain a high-purity bisphenol, the separation of the bisphenol from the phenol by crystallization must be repeated. In addition, it is necessary to separate and recover the phenol as an adduct and to purify the bisphenol by distillation or the like in the end. Thus, the method is very complicated and greatly impairs economic efficiency.

An aromatic polycarbonate is hardly hydrolyzed and almost never hydrolyzed at 230°C or less in the absence of a catalyst ("Chemical Engineering", pp. 689, Sept., 1999).

Meanwhile, it is known that an aromatic polycarbonate is hydrolyzed to form bisphenol A in the absence of a catalyst at a high temperature and a high pressure near the critical point of water. It is considered that it is extremely difficult to carry out the hydrolysis of an aromatic polycarbonate economically because a device and facility become bulky due to the super strong acidity of water itself under the critical water condition in addition to a temperature higher than 300°C and a pressure higher than 200 atm. Hydrolysis using water has a defect that a carbonyl component which is one of the monomer components of a polycarbonate is lost as carbon dioxide.

To cope with this, attempts are being made to recover the carbonyl component as a carbonic acid ester of a monohydric alcohol by depolymerization using the monohydric alcohol. JP-B 6-4549 proposes a method of depolymerizing an aromatic polycarbonate in a polar neutral solvent in the presence of an alkoxide or hydroxide catalyst. JP-A 5-255153 discloses a method of reacting an aromatic polycarbonate with an alcohol in the presence of o-dichlorobenzene as a solvent and a tin compound as a catalyst in a distillation column. In these methods, depolymerization is carried out at a relatively low temperature without using a pressure reactor and the carbonyl component is obtained as a carbonic acid ester such as dimethyl carbonate together with a bisphenol at the same time. However, it is considered that a catalyst is needed and a higher reaction rate is required to carry out depolymerization economically.

The depolymerization reaction of an aromatic polycarbonate using an aliphatic monohydric alcohol proceeds more easily than a hydrolytic reaction using water, the carbonyl component is recovered as a dialkyl carbonate, and the possibility of a reverse reaction (polymerization reaction) which is seen in depolymerization using a phenol is low. Therefore, the above method is considered as one of the most preferred depolymerization methods.

However, as described above, the depolymerization of an aromatic polycarbonate using a monohydric alcohol is mainly carried out in the presence of a solvent and a catalyst at normal pressure or a low pressure and a sufficiently high reaction rate is not obtained. In addition, the method cannot be carried out on an industrial scale because an azeotropic mixture of the monohydric alcohol and the formed dialkyl carbonate is formed and the separation and purification of these components are difficult.

### Summary of the Invention

It is an object of the present invention to provide a novel method of depolymerizing an aromatic polycarbonate.

It is another object of the present invention to provide a method of separating and recovering a carbonyl group as a carbonate of a monohydroxy compound together with an aromatic bisphenol from an aromatic polycarbonate industrially efficiently and economically.

It is still another object of the present invention to provide a depolymerization method capable of depolymerizing an aromatic polycarbonate at a high reaction rate and recovering the above effective components formed by depolymerization at a high rate.

It is a further object of the present invention to provide a depolymerization method capable of recovering and recycling the above effective components from a scrapped or used aromatic polycarbonate.

Other objects and advantages of the present invention will become apparent from the following description.

According to the present invention, firstly, the above objects and advantages of the present invention are attained by a method for depolymerization of an aromatic polycarbonate (may be referred to as "first depolymerization method" hereinafter) which comprises reacting an aromatic polycarbonate with at least one hydroxy compound selected from the group consisting of water, an aliphatic alcohol having 1 to 6 carbon atoms and a phenol which may be substituted by a hydrocarbon group having 1 to 10 carbon atoms in the critical fluid of carbon dioxide.

According to the present invention, secondly, the above objects and advantages of the present invention are attained by a method for depolymerization of an aromatic polycarbonate (may be referred to as "second depolymerization method" hereinafter) which comprises reacting an aromatic polycarbonate with at least one critical fluid selected from the group consisting of an aliphatic alcohol having 1 to 6 carbon atoms and a phenol which may be substituted by a hydrocarbon group having 1 to 10 carbon atoms.

### Brief Description of the Drawing

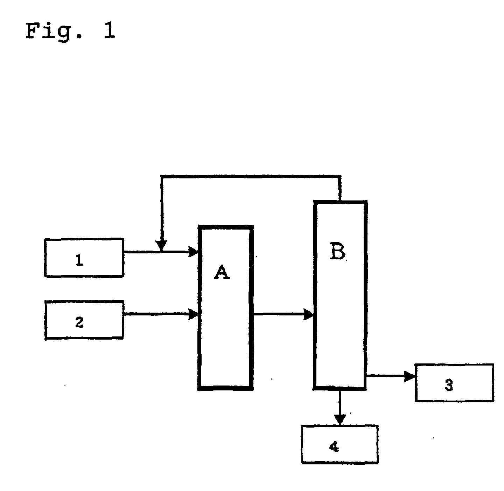

Fig. 1 is a diagram for explaining the steps of a method which is carried out in Example 1.

### The Preferred Embodiment of the Invention

The present invention will be described in detail hereinunder. A description is first given of the first depolymerization method.

The aromatic polycarbonate to be depolymerized in the present invention comprises a carbonic acid ester of an aromatic bisphenol as a recurring unit. Examples of the aromatic bisphenol include dihydroxy benzene, dihydroxybiphenyl, dihydroxydiphenyl ether, dihydroxydiphenyl sulfide, dihydroxydiphenyl sulfone, bis(4-hydroxyphenyl)methane (bisphenol F), 1,1-bis(4-hydroxyphenyl)ethane (bisphenol E), 2,2-bis(4-hydroxyphenyl)propane (bisphenol A), 2,2-bis(4-hydroxy-3-methylphenyl)propane (bisphenol C), 1,1-bis(4-hydroxyphenyl)cyclohexane (bisphenol Z), 3,3,5-trimethyl-1,1-bis(4-hydroxyphenyl)cyclahexane, α,α'-bis(4-hydroxyphenyl)diisopropylbenzene (bisphenol M), 9,9-bis(4-hydroxyphenyl)fluorene, 9,9-bis{(4-hydroxy-3-methyl)phenyl}fluorene, 1,3-bis(4-hydroxyphenyl)-5,7-dimethyladamantane, 2,2-bis(4-hydroxyphenyl)adamantane, 1,3-bis(4-hydroxyphenyl)-p-menthane (YP-90), 2,8-bis(4-hydroxyphenyl)-p-menthane, 1,8-bis(4-hydroxyphenyl)-p-menthane and bisphenol mixtures thereof. Out of these, bisphenol A, bisphenol Z, bisphenol M, 3,3,5-trimethyl-1,1-bis(4-hydroxyphenyl)cyclohexane, 9,9-bis{(4-hydroxy-3-methyl)phenyl}fluorene and YP-90 are preferred, and bisphenol A is particularly preferred.

The aromatic polycarbonate to be depolymerized in the present invention is obtained by polymerizing or copolymerizing the above bisphenol, and any polymerization method is used. In general, either the interfacial polymerization method using phosgene or the melt polymerization method using diphenyl carbonate may be used, and the present invention is not limited to these methods.

The degree of polymerization which differs according to application is generally 5,000 to 200,000. The present invention is not limited to the above range if the aromatic polycarbonate is thermoplastic. Since an aromatic polycarbonate which becomes unnecessary and scrapped is depolymerized in the present invention, it may contain impurities according to application. Therefore, these impurities are preferably removed by a predetermined method in advance. For example, in the case of a scrapped optical disk which has a metal compound recording material on a polycarbonate resin substrate as a thin film, the metal compound recording material can be removed by alkali cleaning or the like after grinding as described in JP-A 7-256639. When an aromatic polycarbonate contains insoluble contaminants, they are removed by filtration or the like after molten.

In the first depolymerization method of the present invention, an aromatic polycarbonate is reacted with at least one hydroxy compound selected from the group consisting of water, an aliphatic alcohol having 1 to 6 carbon atoms and a phenol which may be substituted by a hydrocarbon group having 1 to 10 carbon atoms in the critical fluid of carbon dioxide.

This reaction can be carried out without adding a catalyst to a reaction system.

### (i) A reaction using water as the hydroxy compound can be represented by the following formula when the aromatic polycarbonate is a bisphenol A-based polycarbonate:

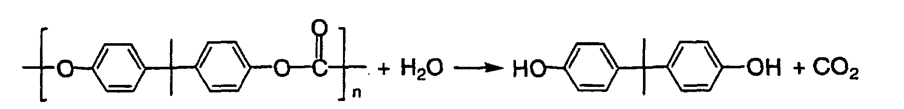

wherein n is an integer of 20 to 800.

The carbon dioxide itself is formed in a stoichiometric amount as the reaction product and its existence and introduction do not cause any contamination. It is extremely easily separated from the bisphenol and can be recycled.

The critical point of carbon dioxide is at a temperature Tc of 31.1°C and a pressure Pc of 7.38 MPa. At a temperature higher than the critical temperature, a critical state that it is not liquefied is maintained at a high pressure. The critical point of water is at an extremely high temperature Tc of 374.2°C and at an extremely high pressure Pc of 21.8 MPa. It is known that the movement and diffusion of a material are high in a critical fluid having a density close to that of a liquid like a low-density gas and the dielectric constant thereof becomes small, thereby improving dissolving power and chemical reactivity. However, when water is used in the hydrolysis (depolymerization) of an aromatic polycarbonate, though the reaction proceeds at a high rate in critical water, it requires extremely high temperature and pressure and the critical water shows super high acidity, whereby an economic burden is large and practical utility is low. Further, a higher-order side-reaction also readily occurs due to the super high acidity of the critical water. Since the aromatic polycarbonate is chemically inactive in the critical fluid of carbon dioxide and the crystallization of the aromatic polycarbonate is promoted, the effect of the critical fluid of carbon dioxide as a medium for a depolymerization reaction is apprehended. The inventors of the present invention have studied the effect in detail and have found that the reaction proceeds in the critical fluid of carbon dioxide at a higher rate than a depolymerization reaction using water alone at the same temperature without a catalyst.

In the first depolymerization method, the aromatic polycarbonate is generally supplied into a reactor after it is ground. It may be supplied after it is molten by heating when the reaction is to be carried out continuously.

The critical fluid of carbon dioxide can be formed by introducing carbon dioxide into a pressure vessel containing water and an aromatic polycarbonate to be reacted with each other under pressure while stirring and by heating it. Further, a hydrolytic reaction is carried out by maintaining a fixed pressure at a fixed temperature using a pressure control valve at the outlet of the reactor to maintain the density of the critical fluid of carbon dioxide at a constant value, and a product which is soluble in the critical fluid and formed by the reaction can be taken out from the outlet of the pressure control valve continuously.

As for the simple method of introducing carbon dioxide, a predetermined amount of solid carbon dioxide (dry ice) is fed to the pressure vessel, and the pressure vessel is sealed up and heated to form a critical fluid so that the reaction can be carried out therein. Also in this case, the reaction can be carried out in the critical fluid having a desired density by controlling the amount of carbon dioxide.

Water as a reactant may be introduced into the reactor together with the aromatic polycarbonate. However, when the reaction is to be carried out continuously, the raw material aromatic polycarbonate can be easily introduced under pressure continuously like the method in which the aromatic polycarbonate is introduced after it is molten and like carbon dioxide. That is, the raw material polymer, water and carbon dioxide are continuously supplied into the reactor under reaction conditions to carry out the depolymerization reaction of the present invention continuously. The amount of water as a reactant is 1 mol or more, preferably 2 mols or more, more preferably 5 mols or more based on 1 mol of the recurring unit of the aromatic polycarbonate. The upper limit of the amount is not particularly limited but 50 mols or less, preferably 30 mols or less based on 1 mol of the recurring unit of the aromatic polycarbonate so that the most of the reaction volume is not filled with liquid phase under the reaction conditions. A too large amount of water is economically unpreferred when depolymerization is carried out on an industrial scale because of its large latent heat.

The reaction of the first depolymerization method is carried out at preferably 200° C or more, more preferably 220°C or more. When the reaction temperature is lower than 200°C, the reaction becomes slow and it takes long to complete the reaction. The upper limit of reaction temperature is 350°C, preferably 300° C. The main reason for this is the durability and safety of the reactor. At a temperature higher than 300° C, the formed bisphenol may be decomposed into phenol or the like disadvantageously.

The pressure of the reaction system is preferably 7.0 MPa or more, more preferably 7.3 to 35 MPa, particularly preferably 8 to 25 MPa.

The depolymerization reaction of the aromatic polycarbonate in the critical fluid of carbon dioxide is generally carried out without a catalyst but may be carried out in the presence of a catalyst to accelerate the reaction rate and increase selectivity. Catalysts used for the hydrolysis of an ester or an ester exchange reaction are used as the catalyst. Examples of the catalyst include hydroxides and salts of alkali metals and alkali earth metals, and oxides and salts of zinc, germanium, tin, lead, titanium, zirconium, antimony and rare earth metals.

An aromatic dihydroxy compound (aromatic bisphenol) which is a product formed after the reaction and a monomer of the aromatic polycarbonate is separated as follows, for example, after the depolymerization reaction of the present invention and purified, thus making possible recycling from waste which is the object of the present invention.

When bisphenol is selectively dissolved in the critical fluid of carbon dioxide as described above, an unreacted polymer and oligomer do not dissolve in the fluid and are phase separated. Therefore, they can be separated by extracting a carbon dioxide phase continuously. After the end of the reaction, the reaction system is opened to remove carbon dioxide therefrom and the reaction product which contains an aromatic bisphenol can be separated. This reaction product containing an aromatic bisphenol is subjected to distillation or extraction with an organic solvent to isolate the aromatic bisphenol. Examples of the organic solvent include alcohols such as methanol, ethanol and isopropanol, ethers such as diethyl ether, and halogen solvents such as chloroform and chlorobenzene.

After the depolymerization reaction of the present invention, a bisphenol of interest may be obtained through crystallization by cooling the reaction product directly or further adding a suitable solvent. The present invention can be used as a separation/purification method. This method is effective according to the types of impurities.

Recycling of the bisphenol may also be accomplished by extracting and separating the bisphenol from the reaction product using a solvent which selectively dissolves the bisphenol and purifying the separated bisphenol by distillation, crystallization or the like. This solvent is identical to the above organic solvent.

### (ii) A reaction using an aliphatic alcohol having 1 to 6 carbon atoms as the hydroxy compound can be expressed by the following formula when the aromatic polycarbonate is a bisphenol A-based polycarbonate:

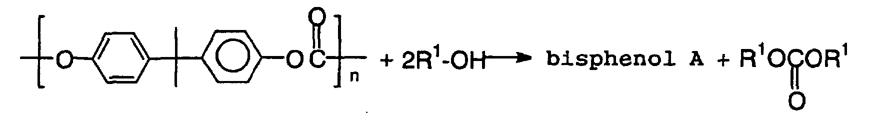

wherein R¹ is an alkyl group having 1 to 6 carbon atoms, and n is an integer of 20 to 800.

This reaction is carried out by reacting the aromatic polycarbonate with the aliphatic alcohol having 1 to 6 carbon atoms in the critical fluid of carbon dioxide at a temperature of 150°C or more to produce an aromatic bisphenol and a di (alkyl having 1 to 6 carbon atoms) carbonate which are constituent components of the aromatic polycarbonate.

Examples of the aliphatic alcohol having 1 to 6 carbon atoms include methanol, ethanol, propanol, butanol and hexanol. Out of these, methanol and ethanol are preferred, and methanol is the most preferred.

The amount of the aliphatic alcohol having 1 to 6 carbon atoms is preferably 1 to 500 mols, more preferably 2 to 100 mols, particularly preferably 5 to 20 mols based on 1 mol of the recurring unit of the aromatic polycarbonate.

The depolymerization reaction of the aromatic polycarbonate in the critical fluid of carbon dioxide is carried out by reacting the aromatic polycarbonate with the aliphatic alcohol under pressure at a temperature of 150°C or more. It is carried out at the above temperature and a pressure close to the steam pressure of an alcohol using a closed vessel, preferably a reaction temperature of 200°C or more, more preferably 150°C or more and 350°C or less. When the reaction temperature is lower than 150°C, the reaction becomes slow and it takes long to complete the reaction disadvantageously. At a temperature higher than 350° C, a side reaction such as alkylation may occur disadvantageously. The reaction temperature is particularly preferably 220 to 280° C, including the critical temperature of an alcohol.

The pressure of the reaction system is preferably 7.0 MPa or more, more preferably 7.3 to 35 MPa, particularly preferably 8 to 25 MPa.

After the end of the reaction, the aromatic bisphenol and the di(alkyl having 1 to 6 carbon atoms) carbonate can be separated from the reaction system by extracting a carbon dioxide phase continuously because the reaction product containing the aromatic bisphenol and the di(alkyl having 1 to 6 carbon atoms) carbonate is selectively dissolved in the critical fluid of carbon dioxide and an unreacted polymer and oligomer do not dissolve in the fluid and are phase separated after the end of the reaction.

The reaction product containing the aromatic bisphenol and the di(alkyl having 1 to 6 carbon atoms) carbonate is subjected to distillation to remove an alcohol having 1 to 6 carbon atoms and the residue after distillation is further subjected to distillation or extraction with an organic solvent to isolate the aromatic bisphenol and the di(alkyl having 1 to 6 carbon atoms) carbonate.

Examples of the formed di(alkyl having 1 to 6 carbon atoms) carbonate include dimethyl carbonate and diethyl carbonate.

When distillation is carried out, any distillation device may be used but a device having a small amount of residence and a short time of residence is used to reduce cost and prevent deterioration. For example, a dropping liquid film evaporator, thin film evaporator, spiral tube evaporator, or circulating or rising film evaporator is preferably used. The residue after distillation may be directly used in the depolymerization reaction of an aromatic polycarbonate.

Examples of the organic solvent are identical to those of the organic solvent in (i).

As for what is not described herein, it should be understood that the above description in (i) is applied directly or with modifications obvious to one of ordinary skill in the art.

### (iii) Further, a reaction using a phenol which may be substituted by a hydrocarbon group having 1 to 10 carbon atoms as the hydroxy compound can be represented by the following formula when the aromatic polycarbonate is a bisphenol A-based polycarbonate:

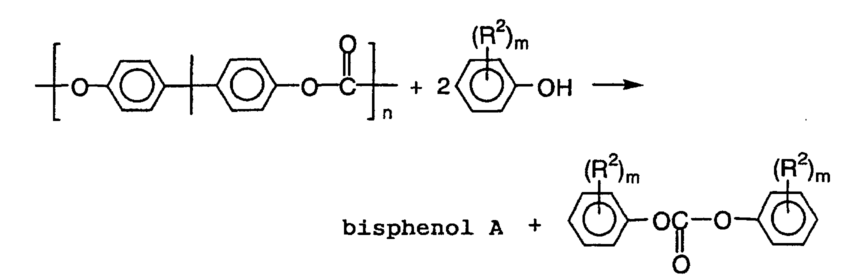

wherein R² is a hydrocarbon group having 1 to 10 carbon atoms, m is an integer of 0 to 5, and n is an integer of 20 to 800.

This reaction is carried out by reacting the aromatic polycarbonate with the phenol which may be substituted by a hydrocarbon group having 1 to 10 carbon atoms in the critical fluid of carbon dioxide at a temperature of 150°C or more to form an aromatic bisphenol and a di(phenyl which may be substituted by a hydrocarbon group having 1 to 10 carbon atoms) carbonate which are the constituent components of the aromatic polycarbonate.

Examples of the hydrocarbon group having 1 to 10 carbon atoms which may substitute the phenol include alkyl groups such as methyl, ethyl, propyl, butyl, hexyl, octyl and decyl.

Examples of the phenol include phenol, orthocresol, metacresol, paracresol, xylenol and isopropylphenol. Out of these, phenol is particularly preferred.

The phenol is used in an amount of preferably 1 to 500 mols, more preferably 2 to 100 mols, particularly preferably 5 to 20 mols based on 1 mol of the recurring unit of the aromatic polycarbonate.

The depolymerization reaction of the aromatic polycarbonate is carried out by reacting the aromatic polycarbonate with the above phenol at a temperature of 150° C or more under pressure. The reaction temperature is preferably 150 to 350°C, more preferably 200 to 300°C.

The reaction pressure is preferably 7.0 MPa or more, more preferably 7.3 to 35 MPa, particularly preferably 8 to 25 MPa.

After the end of the reaction, the aromatic bisphenol and the di(phenyl which may be substituted by a hydrocarbon group having 1 to 10 carbon atoms) carbonate (may be referred to as "diaryl carbonate" hereinafter) can be separated from the reaction system by extracting a carbon dioxide phase continuously because the reaction product containing the aromatic bisphenol and the diaryl carbonate is selectively dissolved in the critical fluid of carbon dioxide and an unreacted polymer and oligomer do not dissolve in the fluid and are phase separated.

The reaction product containing the aromatic bisphenol and the diaryl carbonate is subjected to distillation to remove a phenol which may be substituted by a hydrocarbon group having 1 to 10 carbon atoms and the residue after distillation is further subjected to distillation or extraction with an organic solvent to isolate the aromatic bisphenol and the diaryl carbonate, respectively.

Examples of the formed diaryl carbonate include diphenyl carbonate, ditolyl carbonate and dixylyl carbonate.

Preferably, extraction of the above product with an organic solvent is carried out using an organic solvent having a solubility parameter (δₛ) of 10.5 or less to isolate the aromatic bisphenol from the extraction solution by crystallization, and further the mother liquor is subjected to distillation or an organic solvent having a solubility parameter (δₛ) of 13.0 or more is added to the mother liquor to isolate the diaryl carbonate.

The solubility parameter (δₛ) is a value obtained from K.L. Hoy's concept of mol traction force (J. Paint Technol., 42, 76, 1970).

Organic solvents having a δₛ of 10.5 or less include aromatic hydrocarbon solvents such as benzene (δₛ; 9.1), toluene (δₛ; 8.8) and xylenes (δₛ; 8.5 to 8.8), halogenated hydrocarbons such as methylene chloride and chloroform (δₛ; 9.2), carbon tetrachloride (δₛ; 8.6) and chlorobenzene (δₛ; 9.3), and saturated aliphatic hydrocarbon compounds such as hexane (δₛ; 7.32), heptane (δₛ; 7.44) and pentane (δₛ; 7.37). Out of these, organic solvents having a δₛ of 10.0 to 8.0 are preferred. These organic solvents dissolve an aromatic bisphenol and are excellent in crystallization selectivity.

Out of the above solvents having a solubility parameter (δₛ) of 10.5 or less, solvents having a relatively low boiling point which are chemically stable and easily recovered and recycled and from which predicted impurities can be removed without contaminating the finally targeted materials are the most preferred, and benzene, toluene, methylene chloride and chloroform are particularly preferred. The amount of the solvent is determined by the solubility for an aromatic bisphenol and diaryl carbonate of the solvent and the amounts of a decomposed aromatic polycarbonate (oligomer) and the residual phenol. A solvent having low solubility for an aromatic bisphenol (for example, solubility in 100 g of the solvent at room temperature is 1 or less) and relatively high solubility for a diaryl carbonate (for example, solubility in 100 g of the solvent at room temperature is 10 or more) is selected as the solvent having a solubility parameter δₛ of 10.5 or less used herein. The solvent is used in an amount that the diaryl carbonate can retain its solubility. Therefore, the amount of the solvent actually used which differs according to the solubility (solubility difference) of the solvent, the treating temperature (difference) and the total amount of impurities is 50 to 200 parts by weight based on 100 parts by weight of the above reaction product when toluene is used. Crystallization may be carried out by general means and an aromatic bisphenol having higher purity can be separated and recovered by repeating crystallization and supplied to the next step together with the mother liquor.

After the aromatic bisphenol which is one of the monomer components is separated and recovered, the mother liquor is subjected to distillation or crystallization using a solvent having a solubility parameter δₛ of 13.0 or more to separate and recover the diaryl carbonate. That is, in this step, the diaryl carbonate is separated and recovered by evaporating and collecting the solvent used in the previous step and subjecting the residue to distillation in high vacuum or by adding a solvent having a solubility parameter δₛ of 13.0 or more to the residue for crystallization.

In the case of distillation, any distillation device may be used but a device having a small amount of residence and a short time of residence is used to reduce cost and prevent deterioration. For example, a dropping liquid film evaporator, thin film evaporator, spiral tube evaporator, or circulating or rising film evaporator is preferred. The residue after distillation can be directly used in the depolymerization reaction of the aromatic polycarbonate.

When separation by crystallization is carried out using a solvent having a solubility parameter δₛ of 13.0 or more, methanol or ethanol is preferably used as the solvent. Methanol is particularly preferred. The solubility in methanol of bisphenol A is 409 parts by weight based on 100 parts by weight of methanol at room temperature and the solubility in methanol of diphenyl carbonate is only 10 parts by weight. Therefore, the amount of the solvent used for crystallization in this step is relatively smaller than that of the solvent used in the previous step. The mother liquor after crystallization can be used in the depolymerization reaction of the aromatic polycarbonate or as the solvent in the previous step after methanol is completely removed therefrom.

A description is subsequently given of the second depolymerization method.

The aromatic polycarbonate to be depolymerized is identical to the aromatic polycarbonate in the first depolymerization method.

In the second depolymerization method of the present invention, the aromatic polycarbonate is reacted with at least one critical fluid selected from the group consisting of an aliphatic alcohol having 1 to 6 carbon atoms and a phenol which may be substituted by a hydrocarbon group having 1 to 10 carbon atoms.

Examples of the aliphatic alcohol having 1 to 6 carbon atoms and the phenol which may be substituted by a hydrocarbon group having 1 to 10 carbon atoms are the same as those enumerated in the first depolymerization method.

In the second depolymerization method, the aromatic polycarbonate is reacted with the critical fluid of at least one compound selected from the aliphatic alcohol and the phenol.

These compounds have the following critical points. The critical temperature (Tc, °K) and the critical pressure (Pc, MPa) are given after the names of the compounds. Methanol 512.6, 8.09; ethanol 516.2, 6.38; n-propanol 536.7, 5.17; isopropanol 508.3, 4.76; n-butanol 562.9, 4.42; sec-butanol 536.0, 4.19; iso-butanol 547.7, 4.30; tert-butanol 506.2, 3.97; n-pentanol 586.0, 3.85; 2-methyl-1-butanol 571.0, 3.85; 3-methyl-1-butanol 579.5, 3.85; 2-methyl-2-butanol 545.0, 3.95; 2,2-dimethyl-1-propanol 549.0, 3.95; n-hexanol 610.0, 4.05; cyclohexanol 625.0, 3.75; phenol 694.2, 6.13; o-cresol 697.6, 5.00; m-cresol 705.8, 4.56; p-cresol 704.6, 5.15; o-ethylphenol 703.0, 3.42; m-ethylphenol 716.4, 3.42; p-ethylphenol 716.4, 3.42; 2,3-xylenol 722.8, 3.42; 2,4-xylenol 707.6, 3.42; 2,5-xylenol 723.0, 3.42; 2,6-xylenol 701.0, 3.42; 3,4-xylenol 729.8, 3.42; and 3,5-xylenol 715.6, 3.42.

The reaction between the aromatic polycarbonate and the critical fluid of the above compound is identical to the reaction between the aromatic polycarbonate and the same compound in the first depolymerization method.

This reaction is carried out (i) by reacting the aromatic polycarbonate with the critical fluid of an aliphatic alcohol having 1 to 6 carbon atoms to form an aromatic bisphenol and a di(alkyl having 1 to 6 carbon atoms) carbonate which are the constituent components of the aromatic polycarbonate, or (ii) by reacting the aromatic polycarbonate with the critical fluid of a phenol which may be substituted by a hydrocarbon group having 1 to 10 carbon atoms to form an aromatic bisphenol and a di(phenyl which may be substituted by a hydrocarbon group having 1 to 10 carbon atoms) carbonate (diaryl carbonate) which are the constituent components of the aromatic polycarbonate.

All the above reactions (i) and (ii) are carried out at a temperature of (Tc)(temperature at which the aliphatic alcohol or the phenol forms a critical fluid)±30°C and a pressure of (Pc)(pressure at which the aliphatic alcohol or the phenol forms a critical fluid)±2 MPa.

As for the reaction (i), since methanol and ethanol show high dissolving power and high dispersibility at the same time though the densities at the critical point of methanol and ethanol are 0.272 g/cc and 0.276 g/cc which are about 1/3 the densities of methanol and ethanol in a liquid state, the solvent-added decomposition reaction of the aromatic polycarbonate proceeds at an extremely high rate without a catalyst. The reaction conditions close to this critical point are the most advantageous. Under the conditions, the reaction is completed in a very short period of time and the aromatic bisphenol and the dialkyl carbonate which are formed by the reaction are obtained in a dissolved state in the critical fluid of the alcohol.

After the end of the reaction, the dialkyl carbonate and the aromatic bisphenol are acquired by separating from the reaction mixture. For example, the following method is preferably carried out: after the end of the reaction, the aliphatic alcohol having 1 to 6 carbon atoms in the reaction system is made in a non-critical state by reducing the inside pressure of the reaction system and then the reaction product is subjected to distillation so as to distill off the dialkyl (1 to 6 carbon atoms) carbonate and acquire the aromatic bisphenol as the residue while the aliphatic alcohol having 1 to 6 carbon atoms is distilled off as an azeotropic mixture with the di(alkyl having 1 to 6 carbon atoms) carbonate.

In order to obtain the dialkyl carbonate separate from the azeotropic mixture as much as possible, the azeotropic mixture preferably contains a small amount of the dialkyl carbonate.

Preferably, the azeotropic mixture comprises the aliphatic alcohol having 1 to 6 carbon atoms and the di(alkyl having 1 to 6 carbon atoms) carbonate in a weight ratio of 70:30 to 99:1.

When distillation is carried out under pressure, the composition of the azeotropic mixture changes. As the pressure becomes higher, the content of a low-boiling point component in the azeotropic mixture tends to increase.

When the aliphatic alcohol is methanol, an azeotropic mixture comprising methanol and dimethyl carbonate in a weight ratio of about 93:7 is obtained at 1.5 MPa. Therefore, a methanol fraction having a low content of dimethyl carbonate is obtained from the top of a distillation column by distillation and separation at a high pressure and dimethyl carbonate can be distilled out from the lower portion of the column. Simultaneously, an aromatic bisphenol such as bisphenol A having the highest boiling point can be obtained from the bottom of the distillation column. Further, the bisphenol A can be further purified by distillation or crystallization as required. The methanol fraction obtained from the top of the column contains a small amount of dimethyl carbonate as a component of the azeotropic mixture and is recycled to the depolymerization reactor.

Accordingly, as the content of the dialkyl carbonate in the alcohol distilling out from the top of the column becomes smaller as the operation pressure of the distillation column increases, the distillation column is preferably operated at a higher pressure. For instance, when methanol is used as the alcohol, the ratio of methanol to dimethyl carbonate is about 93/7 at 1.5 MPa, about 96/4 at 2.0 MPa and about 98.5/1.5 at 3.0 MPa. Thus, the amount of dimethyl carbonate recycled together with methanol is small and the ratio of dimethyl carbonate acquired from the lower portion of the distillation column improves.

Since the depolymerization reaction is carried out at a high temperature under pressure as described above, the operation pressure of the distillation column is preferably lower than the reaction pressure of the depolymerization reaction and as high as possible to improve energy efficiency as a whole. Further, as the distillation temperature of the dialkyl carbonate which distills out from the lower portion of the column is determined by the operation pressure of the distillation column, the operation pressure of the distillation column is preferably selected from the viewpoint of total energy efficiency to ensure that that the temperature becomes lower than the temperature of the depolymerization reaction. For example, the vapor pressure at 200°C of dimethyl carbonate is about 1.4 MPa and that at 250° C is about 3.1 MPa. Therefore, the operation pressure of the distillation column is preferably lower than the vapor pressure of the dialkyl carbonate at the temperature of the depolymerization reaction, generally 4.0 MPa or less.

As for (ii), after the end of the reaction, the aromatic bisphenol and the diaryl carbonate are acquired by separating from the reaction mixture.

For example, the following method is preferably carried out: after the end of the reaction, the phenol which may be substituted by a hydrocarbon group having 1 to 10 carbon atoms in the reaction system is made in a non-critical state by reducing the inside pressure of the reaction system, the reaction product is subjected to distillation to distill off the phenol which may be substituted by a hydrocarbon group having 1 to 10 carbon atoms, the residue after distillation is crystallized with an organic solvent having a solubility parameter (δₛ) of 10.5 or less to isolate the aromatic bisphenol, and further the mother liquor is subjected to distillation or an organic solvent having a solubility parameter (δₛ) of 13.0 or more is added to the mother liquor so as to isolate the diaryl carbonate.

Distillation may be carried out under pressure or without pressure.

The distillation temperature is, for example, 100 to 400°C.

The crystallization of the residue after distillation, the distillation of the mother liquor and the used organic solvent are the same as in the first depolymerization method.

According to the present invention, there is provided a method of isolating useful substances such as an aromatic bisphenol and a diaryl carbonate from the depolymerized product of an aromatic polycarbonate as described above.

This method is preferably carried out by depolymerizing the aromatic polycarbonate with phenol, tert-butylphenol or cumylphenol to obtain a reaction product containing an aromatic bisphenol and a diaryl carbonate, (i) subjecting this reaction product to distillation to remove phenol, tert-butylphenol or cumylphenol and (ii) crystallizing the residue after distillation with an organic solvent having a solubility parameter (δₛ) of 10.5 or less to isolate the aromatic bisphenol and subjecting the mother liquor to distillation or adding an organic solvent having a solubility parameter (δₛ) of 13.0 or more to the mother liquor to isolate the diaryl carbonate.

As for what is not described of the second depolymerization method and the above method, it should be understood that the description of the first depolymerization method is applied directly or with modifications obvious to one of ordinary skill in the art.

### Examples

The following examples are provided for the purpose of further illustrating the present invention but are in no way to be taken as limiting.

### Example 1

5.0 g of polycarbonate resin pellets (AD-5503 of Teijin Chemicals, Ltd., average molecular weight of 15,000) and 10 ml of water were fed to an autoclave having a total capacity of 113 ml, 15 g of solid carbon dioxide (dry ice) was fed to the autoclave, and the autoclave was sealed up and heated under agitation with a magnetic stirrer. After the temperature reached 250°C, that temperature was maintained for 4 hours. The pressure was 228 atm. After the reaction, the autoclave was cooled to room temperature quickly, carbon dioxide was discharged, and the contents of the autoclave were taken out. When the contents were analyzed by gas chromatography, 3.49 g of bisphenol A was obtained (yield of 78 %). The product was all soluble in methanol and the raw material polymer was almost inexistent.

### Example 2

Like Example 1, 5.0 g of polycarbonate resin pellets, 10 ml of water and 25 g of carbon dioxide were fed to an autoclave having a total capacity of 113 ml, stirred while the autoclave was sealed up, heated at 230°C and maintained at that temperature for 4 hours. The pressure was 202 atm. After the reaction, the autoclave was cooled to discharge carbon dioxide and the contents were taken out and divided into a methanol-soluble portion and methanol-insoluble portion. When the methanol-soluble portion was analyzed by gas chromatography, 0.77 g of bisphenol A was obtained (yield of 16.5 %). The methanol-insoluble portion was dried and when 4.02 g of the portion was used for the measurement of viscosity in E sol, the viscosity ηsp/c was 0.171. (ηsp/c of the original polycarbonate was 0.47 which was reduced by depolymerization.)

### Comparative Example 1

Like Example 2, 5.0 g of polycarbonate resin pellets and 10 ml of water were fed to a 113 ml autoclave, stirred while the autoclave was sealed up, heated at 230°C and maintained at that temperature for 4 hours. After the reaction, the autoclave was cooled and its contents were taken out. The polycarbonate pellets almost remained unchanged and the formation of bisphenol A was not observed. When the solution viscosity of the collected polymer was measured, ηsp/c was 0.417 which means that depolymerization proceeded just a little. (ηsp/c of the original polycarbonate was 0.47.)

### Example 3 (reaction in critical methanol)

The method carried out in Example 3 is shown in the process diagram of the attached Fig. 1. 5.0 g of polycarbonate resin 2 (ground product of a compact disk) was fed to a 113 ml autoclave A and heated at 245°C under agitation with a magnetic stirrer. When the temperature reached 245°C, preheated methanol was continuously added to the autoclave at a rate of 2.5 ml/min to carry out a reaction. After 30 minutes, the introduction of methanol was stopped to complete the reaction. The pressure at this point was 8.3 MPa. The reaction product was then transferred to a pressure distillation column B and subjected to distillation at 150°C and 1.5 MPa, an azeotropic mixture obtained from the top of the column was returned to the autoclave A together with methanol, dimethyl carbonate 3 was taken out from the middle portion of the column, and bisphenol A4 was taken out from the bottom of the column. When the quenched contents taken out from the autoclave A was analyzed by gas chromatography, it was found that 1.6 g of dimethyl carbonate and 4.1 g (yield of 92 %) of bisphenol A were formed.

### Example 4 (reaction in subcritical methanol)

Like Example 3, 5.0 g of a polycarbonate resin (ground product of a compact disk whose deposited product on the surface has been removed: for example, polycarbonate resin disclosed by JP-A 7-256639) was fed to a 113 ml autoclave and heated under agitation with a magnetic stirrer. When the temperature reached 235°C, preheated methanol was continuously introduced into the autoclave at a rate of 2.5 ml/min to carry out a reaction. After 30 minutes, the introduction of methanol was stopped to complete the reaction. The pressure at this point was 6.6 MPa. After the reaction, the autoclave was cooled to room temperature quickly, and then its contents were taken out. When the contents were analyzed by gas chromatography, it was found that 1.7 g of dimethyl carbonate and 4.2 g (yield of 93 %) of bisphenol A were formed.

### Example 5 (critical CO₂ + methanol)

5.0 g of a polycarbonate resin (ground product of a compact disk whose deposited product on the surface has been removed: for example, polycarbonate resin disclosed by JP-A 7-256639) and 20 g of methanol were fed to a 113 ml autoclave, and the autoclave was sealed up. The autoclave was heated under agitation with a magnetic stirrer and a valve at the inlet was opened while the temperature was raised to introduce liquid carbon dioxide until a predetermined reaction temperature of 200°C and a predetermined reaction pressure of 20 MPa were achieved. After the predetermined temperature and pressure were reached, they were maintained for 1 hour. After the reaction, the autoclave was cooled to room temperature quickly, and then its contents were taken out. When the contents were analyzed by gas chromatography, it was found that 1.6 g of dimethyl carbonate and 3.9 g (yield of 87 %) of bisphenol A were formed.

### Example 6

128 g of polycarbonate resin pellets (AD-5503 of Teijin Chemicals, Ltd., average molecular weight of 15,000) and 380 ml of phenol were fed to a 500 ml autoclave and heated under agitation with a magnetic stirrer. After the temperature reached 250°C, that temperature was maintained for 4 hours. The pressure was 0.1 MPa. After the reaction, the autoclave was cooled to room temperature quickly, and then its contents were taken out. When the contents were analyzed by gas chromatography, it was found that 104.3 g of bisphenol A, 96.7 g (yield of 90 %) of diphenyl carbonate and 299 g of phenol were contained.

This reaction mixture was transferred to a 1,000 ml flask which was then mounted on a rotary evaporator on a water bath for vacuum evaporation. About 280 g of phenol was recovered at a water bath temperature of 90°C and a vacuum degree of 3 mmHg. After the phenol was distilled out almost completely, 200 ml of toluene was added, stirred for about 10 minutes under heating and left to stand at room temperature.

Crystals were separated by filtration and cleaned with a small amount (about 30 ml) of toluene to obtain about 330 g of a toluene solution consisting of about 105 g of crystals, cleaning liquid and mother liquor. When they were analyzed by gas chromatography, the crystals were bisphenol A having a purity of about 96 % and the toluene solution contained 91 g of diphenyl carbonate and 4.3 g of bisphenol A. About 100 ml of toluene was added to the crystals, stirred for 1 hour, left to stand for several hours and filtered to produce about 99 . 3 g of bisphenol A having a purity of 99 % or more as crystals.

After the above toluene solutions were mixed together to distill off toluene, 100 ml of methanol was added, stirred, left to stand for one night at room temperature and filtered to produce about 91 g of crystals. When the crystals were analyzed by gas chromatography, they were diphenyl carbonate having a purity of about 92 %.

### Example 7

5.0 g of a polycarbonate resin (ground product of a compact disk whose deposited product on the surface has been removed: for example, polycarbonate resin disclosed by JP-A 7-256639) and 20 g of phenol were fed to a 113 ml autoclave and the autoclave was sealed up. The autoclave was heated under agitation with a magnetic stirrer and a valve at the inlet was opened under heating to introduce liquid carbon dioxide until a predetermined reaction temperature of 250°C and a predetermined reaction pressure of 20 MPa were achieved. After the predetermined temperature and pressure were reached, they were maintained for 1 hour. After the reaction, the autoclave was cooled to room temperature quickly, and then its contents were taken out. When the contents were analyzed by gas chromatography, it was found that 3.9 g of diphenyl carbonate and 4.2 g (yield of 93 %) of bisphenol A were formed.

## Claims

1. A method for depolymerization of an aromatic polycarbonate, which comprises reacting an aromatic polycarbonate with at least one hydroxy compound selected from the group consisting of water, an aliphatic alcohol having 1 to 6 carbon atoms and a phenol which may be substituted by a hydrocarbon group having 1 to 10 carbon atoms in the critical fluid of carbon dioxide.

2. The method of claim 1, wherein the reaction is carried out without adding a catalyst to a reaction system.

3. The method of claim 1, wherein the aromatic polycarbonate is reacted with water in the critical fluid of carbon dioxide at a temperature of 200°C or more to form an aromatic bisphenol which is a constituent component of the aromatic polycarbonate.

4. The method of claim 1, wherein the aromatic polycarbonate is reacted with an aliphatic alcohol having 1 to 6 carbon atoms in the critical fluid of carbon dioxide at a temperature of 150°C or more to form an aromatic bisphenol and a di(alkyl having 1 to 6 carbon atoms) carbonate which are the constituent components of the aromatic polycarbonate.

5. The method of claim 1, wherein the aromatic polycarbonate is reacted with a phenol which may be substituted by a hydrocarbon group having 1 to 10 carbon atoms in the critical fluid of carbon dioxide at a temperature of 150°C or more to form an aromatic bisphenol and a di(phenyl which may be substituted by a hydrocarbon group having 1 to 10 carbon atoms) carbonate which are the constituent components of the aromatic polycarbonate.

6. The method of claim 3, wherein carbon dioxide is removed from the reaction system by opening the reaction system and the reaction product containing an aromatic bisphenol is separated after the end of the reaction.

7. The method of claim 4, wherein carbon dioxide is removed from the reaction system and the reaction product containing an aromatic bisphenol and a di (alkyl having 1 to 6 carbon atoms) carbonate is separated after the end of the reaction.

8. The method of claim 5, wherein carbon dioxide is removed from the reaction system by opening the reaction system and the reaction product containing an aromatic bisphenol and a di(phenyl which may be substituted by a hydrocarbon group having 1 to 10 carbon atoms) carbonate is separated after the end of the reaction.

9. The method of claim 6, wherein the reaction product containing an aromatic bisphenol is subjected to distillation or extraction with an organic solvent to isolate the aromatic bisphenol.

10. The method of claim 7, wherein the reaction product containing an aromatic bisphenol and a di(alkyl having 1 to 6 carbon atoms) carbonate is subjected to distillation to remove an alcohol having 1 to 6 carbon atoms and the residue after distillation is further subjected to distillation or extraction with an organic solvent to isolate the aromatic bisphenol and the di(alkyl having 1 to 6 carbon atoms) carbonate.

11. The method of claim 8, wherein the reaction product containing an aromatic bisphenol and a di(phenyl which may be substituted by a hydrocarbon group having 1 to 10 carbon atoms) carbonate is subjected to distillation to remove a phenol which may be substituted by a hydrocarbon group having 1 to 10 carbon atoms and the residue after distillation is further subjected to distillation or extraction with an organic solvent to isolate the aromatic bisphenol and the di(phenyl which may be substituted by a hydrocarbon group having 1 to 10 carbon atoms) carbonate.

12. The method of claim 11, wherein extraction with an organic solvent is carried out using an organic solvent having a solubility parameter (δₛ) of 10.5 or less to isolate the aromatic bisphenol from the extraction solution by crystallization and further the mother liquor is subjected to distillation or an organic solvent having a solubility parameter (δₛ) of 13.0 or more is added to the mother liquor to isolate the di(phenyl which may be substituted by a hydrocarbon group having 1 to 10 carbon atoms) carbonate.

13. A method for depolymerization of an aromatic polycarbonate, which comprises reacting an aromatic polycarbonate with at least one critical fluid selected from the group consisting of an aliphatic alcohol having 1 to 6 carbon atoms and a phenol which may be substituted by a hydrocarbon group having 1 to 10 carbon atoms.

14. The method of claim 13, wherein the aromatic polycarbonate is reacted with the critical fluid of an aliphatic alcohol having 1 to 6 carbon atoms to form an aromatic bisphenol and a di(alkyl having 1 to 6 carbon atoms) carbonate which are the constituent components of the aromatic polycarbonate.

15. The method of claim 13, wherein the aromatic polycarbonate is reacted with the critical fluid of a phenol which may be substituted by a hydrocarbon group having 1 to 10 carbon atoms to form an aromatic bisphenol and a di(phenyl which may be substituted by a hydrocarbon group having 1 to 10 carbon atoms) carbonate which are the constituent components of the aromatic polycarbonate.

16. The method of claim 14, wherein after the end of the reaction, the aliphatic alcohol having 1 to 6 carbon atoms in the reaction system is made in a non-critical state by reducing the inside pressure of the reaction system and then the reaction product is subjected to distillation so as to distill off the dialkyl(1 to 6 carbon atoms) carbonate and acquire the aromatic bisphenol as the residue while the aliphatic alcohol having 1 to 6 carbon atoms is distilled off as an azeotropic mixture with the di(alkyl having 1 to 6 carbon atoms) carbonate.

17. The method of claim 16, wherein the azeotropic mixture contains the aliphatic alcohol having 1 to 6 carbon atoms and the di(alkyl having 1 to 6 carbon atoms) carbonate in a weight ratio of 70:30 to 99:1,

18. The method of claim 16, wherein the aliphatic alcohol having 1 to 6 carbon atoms is methanol and the non-critical state maintains the reaction system under pressure.

19. The method of claim 15, wherein after the end of the reaction, the phenol which may be substituted by a hydrocarbon group having 1 to 10 carbon atoms in the reaction system is made in a non-critical state by reducing the inside pressure of the reaction system, the reaction product is subjected to distillation to distill off the phenol which may be substituted by a hydrocarbon group having 1 to 10 carbon atoms, the residue after distillation is crystallized with an organic solvent having a solubility parameter (δₛ) of 10.5 or less to isolate the aromatic bisphenol, and further the mother liquor is subjected to distillation or an organic solvent having a solubility parameter (δₛ) of 13.0 or more is added to the mother liquor to isolate the di(phenyl which may be substituted by a hydrocarbon group having 1 to 10 carbon atoms) carbonate.

20. A method of isolating useful substances from the depolymerized product of an aromatic polycarbonate, which comprises (i) subjecting a reaction product containing an aromatic bisphenol and a diaryl carbonate obtained by depolymerizing an aromatic polycarbonate with phenol, tert-butylphenol or cumylphenol to distillation to remove the phenol, tert-butylphenol or cumylphenol, (ii) crystallizing the residue after distillation with an organic solvent having a solubility parameter (δₛ) of 10.5 or less to isolate the aromatic bisphenol and further subjecting the mother liquor to distillation or adding an organic solvent having a solubility parameter (δₛ) of 13.0 or more to the mother liquor to isolate the diaryl carbonate.
